# EUROPÄISCHE PATENTSCHRIFT

(11) **EP 1 755 467 B1**
(45) Veröffentlichungstag und Bekanntmachung des Hinweises auf die Patenterteilung: **14.10.2009**
(21) Anmeldenummer: 05749289.4
(22) Anmeldetag: 25.05.2005
(51) Int. Cl.: A61B 17/16

(54) **VORRICHTUNG ZUM AUSSTANZEN VON GEWEBEBEREICHEN AN KNOCHEN**
DEVICE FOR CUTTING OUT TISSUE REGIONS ON BONES
DISPOSITIF POUR DECOUPER DES ZONES TISSULAIRES SUR DES OS

(30) Priorität: 03.06.2004 DE 102004028429
(43) Veröffentlichungstag der Anmeldung: 28.02.2007
(73) Patentinhaber: Karl Storz GmbH & Co. KG, 78532 Tuttlingen (DE)
(72) Erfinder: STEINWACHS, Matthias, 79112 Freiburg (DE); BERBERICH, Sascha, 78532 Tuttlingen (DE)
(74) Vertreter: Weller, Wolfgang
(86) Internationale Anmeldenummer: PCT/EP2005/005624
(87) Internationale Veröffentlichungsnummer: WO 2005/117719

(56) Entgegenhaltungen:
- US-A- 5 782 835
- US-A1- 2003 022 132
- US-A1- 2004 034 437

## Beschreibung

Die Erfindung betrifft eine Vorrichtung zum Ausstanzen von Gewebebereichen an Knochen, mit einer Schneide, deren umlaufende Schneidekante der Kontur eines auszustanzenden Gewebebereiches entspricht.

Eine derartige Vorrichtung ist aus der US 2004/034437 A1 (Basis für den Oberbegriff des Anspruchs 1) bekannt.

Derartige Gewebestanzen, auch Knorpelstanzen genannt, werden von der Firma ARS ARTHRO AG in 73728 Esslingen, Deutschland, vertrieben.

Solche Stanzen werden beispielsweise bei der Autologen Chondrozyten Transplantation (ACT) eingesetzt, siehe DGU - Mitteilungen und Nachrichten 45/2002 1, "ACT und Tissue Engineering" unter Schirmherrschaft der DGU und DGOOC.

Der hyaline Gelenkknorpel des Menschen ist je nach Topografie unterschiedlich dick. Im Bereich der Patella kann er eine Schichtdicke von 7 bis 8 mm erreichen. Da der Gelenkknorpel keinen unmittelbaren Gefäß- oder Nervenanschluss besitzt, erfolgt seine Ernährung überwiegend durch Diffusion aus der Synovialflüssigkeit des Gelenkbinnenraums. Die Vernetzung unterschiedlicher Matrixkomponenten zur Knorpelgrundsubstanz ermöglicht eine mechanische Dämpfung und das fast reibungslose Gleiten der Gelenkflächen. Feingeweblich findet sich ein komplexer Aufbau aus Knorpelzellen (Chondrozyten), Kollagenfasern und Proteoglykanen. Der gesunde hyaline Knorpel eines Erwachsenen ist in der Lage, Belastungskräfte zu tolerieren, die ein Vielfaches des Körpereigengewichtes betragen können.

Verletzungen des Gelenkknorpels stellen ein großes Problem im traumatologisch-orthopädischen Alltag dar. Das eingeschränkte Heilungsvermögen des hyalinen Knorpels ist seit langem bekannt und ist im Wesentlichen in seiner besonderen Struktur und Anatomie begründet.

Ein Schaden an der Gelenkoberfläche, vor allem im Bereich der Belastungszone der Gelenklauffläche, birgt daher das erhöhte Risiko einer weitergehenden Gelenkschädigung im Sinne einer frühzeitigen Arthrose. Bekannte Verfahren zur biologischen Rekonstruktion eines vollschichtigen Knorpelschadens sind meist nur für kleine bis mittlere Defektgrößen geeignet. Bei vollschichtigen Knorpelschäden, vor allem im Bereich des Kniegelenkes, mit mehr als ca. 4 cm² Defektausbreitung findet daher zunehmend die Autologe Chondrozyten Transplantation (ACT) klinische Anwendung.

Bei diesem Verfahren wird eine arthroskopische Entnahme eines Knorpelbiopsats aus einem nichttragenden Gelenkanteil durchgeführt. Die Knorpelzellen aus dem Biopsat werden isoliert, und diese werden in einer Zellkultur angezüchtet.

Die angezüchteten Zellen werden während eines Zweiteingriffs in die Knorpeldefektzone retransplantiert.

In der Knorpeldefektzone wird das defekte Gewebe zuvor entfernt, wozu eine eingangs genannte Vorrichtung zum Ausstanzen eingesetzt wird. Bei dieser so genannten Defektpräparation müssen die Ränder möglichst glatt und im rechten Winkel präpariert werden, und zwar derart, dass die präparierten Defektränder nach allen Seiten von stabilem Knorpel begrenzt werden. Der Defektboden darf bis auf den subchondralen Knochen reichen. Dabei wird eine der Defektgröße entsprechende Knorpelstanze aufgesetzt und fest eingedrückt. Die Kontur der umlaufenden Schneide entspricht dabei etwa der Kontur des auszustanzenden Gewebebereiches, dieser Bereich kann kreisförmig, oval oder anderweitig ausgeformt sein. Mittels eines scharfen Löffels oder einer Ringkürette wird der defekte Gewebebereich bis in die osteochondrale Zone abgeschabt (debridiert).

In den so präparierten Bereich wird dann das zuvor hergestellte Biopsat retransplantiert.

Die bekannten eingesetzten Stanzen weisen eine durchgehende umlaufende Schneidekante auf, die in einer Ebene liegt. Die Stanzen bestehen üblicherweise aus einem Hohlkörper, beispielsweise einem Rohr, dessen etwa senkrecht zur Längsachse verlaufende Endkante zu einer Schneidekante angeschliffen ist.

Im praktischen Einsatz wurde nun festgestellt, dass mit derartigen Schneiden keine optimale Defektpräparation durchgeführt werden kann, da die Knochenfläche, von der der Gewebebereich abgetrennt werden soll, meist nicht eben ist. Wie zuvor erwähnt, treten solche Defekte insbesondere im Bereich des Kniegelenkes auf, bei dem die Knochenoberfläche stark gekrümmt ist.

Das Eintreiben der Knochenstanze mit einer ebenen Schneidelinie auf eine gekrümmte Knochenfläche führt zwangsläufig dazu, dass nur einige Gewebebereiche bis zur optimalen Tiefe eingeschnitten werden können, andere jedoch nicht.

Behelfsmaßnahmen, wie ein Hin- und Herkippen der Stanze, führen nicht zu den gewünschten glatten und im rechten Winkel präparierten Rändern.

Es ist daher Aufgabe der vorliegenden Erfindung, hier Abhilfe zu schaffen und eine Vorrichtung der eingangs genannten Art dahingehend weiterzuentwickeln, dass eine optimale Defektpräparation möglich ist.

Erfindungsgemäß wird die Aufgabe dadurch gelöst, dass die Schneide aus mehreren Schneidensegmenten zusammengesetzt ist, die jeweils in einer axialen Richtung gegen die Kraft eines elastischen Elementes bewegbar sind.

Diese Maßnahmen haben den Vorteil, dass durch die Aufteilung in die einzelnen Schneidensegmente und deren Beaufschlagung jeweils mit der Kraft eines elastischen Elementes diese Schneidensegmente unabhängig von den anderen Schneidensegmenten in das Knorpelgewebe eindringen können. Stellt man sich einen domartig gewölbten Knochenbereich vor, und setzt man die Schneide im Bereich einer Kuppe an, so können in denjenigen Bereichen, in denen die Kuppel schon etwas weiter abgefallen ist, die entsprechenden Schneidensegmente tiefer in das Knorpelgewebe eindringen.

Mit anderen Worten können sich die einzelnen Schneidensegmente, was ihre Eindringtiefe betrifft, an die Kontur der darunter liegenden (subchrondalen) Knochenoberfläche anpassen. Es wird also eine Stanzlinie erzeugt, die sich automatisch an die jeweilige Knochenunterstruktur anpasst. Da die Schneidensegmente in axialer Richtung beweglich sind, entsteht ein Schneiderand, der zuverlässig glatt und entsprechend rechtwinklig ausgebildet ist. Durch die Wahl der Anzahl der Schneidensegmente ist es möglich, eine dem gewellten Untergrund bzw. der Knochenoberfläche mehr oder weniger angepasste Schneidelinie zu schaffen. Ist der Knochengrund nur sanft gewellt oder gekrümmt, kann man sich bei einer Schneidelinienlänge von 10 cm beispielsweise mit 5 oder 10 Schneidensegmenten begnügen, bei einer stark gewellten Oberfläche können entsprechend mehr Segmente, beispielsweise 20 Schneidensegmente, vorgesehen sein.

Man erzielt somit eine optimale Anpassung der Schneidelinie an die Anatomie der Knochenoberfläche, so dass eine optimale Präparation des Defektes möglich ist. Insbesondere ist das anschließende Abschaben des ausgestanzten Gewebebereiches von dem Knochenuntergrund wesentlich einfacher durchzuführen, als wenn einige Bereiche des Gewebes aufgrund der welligen Untergrundgeometrie des Knochens nicht bis zur optimalen Tiefe angeschnitten bzw. angestanzt wären.

Das elastische Element kann aus einem elastisch verformbaren Material bestehen.

In einer Ausgestaltung ist das elastische Element als Feder ausgebildet, gegen deren Kraft ein Schneidesegment bewegt werden kann.

In einer weiteren Ausgestaltung der Erfindung sind die Schneidensegmente in einer Führung aufgenommen.

Diese Maßnahme hat den Vorteil, dass durch die Führung die Schneidensegmente bei ihrer axialen Hin- und Herbewegung exakt geführt sind, so dass besonders glatte und rechtwinklige Schnitte durchgeführt werden können.

In einer weiteren Ausgestaltung der Erfindung ist ein Schneidensegment derart durch das elastische Element bzw. die Feder beaufschlagbar, dass dieses über einen axialen Längenabschnitt über den distalen Rand der Führung herausdrückbar ist.

Diese Maßnahme hat den Vorteil, dass ein variabler Längenabschnitt vorhanden ist, der auch zugleich die maximale Eindringtiefe der Schneide in das Gewebe bestimmt.

Dies ermöglicht nicht nur die variable Anpassung der Schneidelinie an die Kontur der Knochenoberfläche, von der das Knorpelgewebe abgetrennt werden soll, sondern begrenzt auch die maximale Eindringtiefe, so dass eine Verletzung des Knochens durch ein versehentlich zu weites Eintreiben durch die Schneide der Stanze ausgeschlossen wird.

In einer weiteren Ausgestaltung der Erfindung weist die Führung eine äußere Wand und eine radial nach innen davon beabstandete innere Wand auf, zwischen denen die Schneidensegmente aufgenommen sind.

Diese Maßnahme hat den Vorteil, dass die Schneidensegmente in radialer Richtung durch die Wand geführt und auch vor Beschädigungen geschützt werden. Dadurch ist es auch möglich, die einzelnen Schneidensegmente nebeneinander als Einzelsegmente einzusetzen und anzuordnen, da diese durch die innere und äußere Wand gehalten und geführt werden und sie werden in umfänglicher Richtung zusätzlich durch ein benachbartes Schneidensegment geführt.

Dies erlaubt eine besonders funktionssichere Handhabung.

In einer weiteren Ausgestaltung der Erfindung ist die Vorrichtung als Hohlkörper aufgebaut, der in zumindest zwei Hohlkörpersegmente aufgeteilt ist, wobei zumindest ein Hohlkörpersegment gegenüber einem anderen axial verschiebbar ist.

Diese Maßnahme hat den erheblichen Vorteil, dass zunächst der Stanzvorgang durchgeführt werden kann und anschließend ein Hohlkörpersegment axial abgezogen werden kann, wodurch genügend Raum geschaffen ist, um mit dem entsprechenden Löffel das Gewebe vom Knochen abzuschaben. Ein Teil der Schneiden ist noch in das Gewebe eingedrückt, wodurch dieser Bereich als Anlage oder als Anschlag für den Löffel dient, so dass dieses Abschaben so erfolgen kann, dass die Ränder durch diesen Abschabvorgang nicht beschädigt werden.

Dies ergibt besonders exakte und glatte Ränder im ausgestanzten Bereich.

In einer weiteren Ausgestaltung der Erfindung weist ein Hohlkörpersegment die Führung und die von dieser geführten Schneidensegmente auf.

Der Hohlkörper ist also aus verschiedenen Schneideeinheiten aufgebaut, von denen zumindest eine als komplette Baueinheit axial abgezogen werden können, so dass für den Operateur ein ausreichender Raum zur Verfügung steht, um mit dem Löffel die Ausschabvorgänge durchzuführen.

In einer weiteren Ausgestaltung der Erfindung sind die Schneidensegmente eines Hohlkörpersegments zusammen in der Führung axial hin- und her bewegbar.

Diese Maßnahme hat den erheblichen Vorteil, dass die Schneidensegmente des Hohlkörpersegments, das abgezogen wird, zusätzlich noch weiter axial zurückgezogen werden können, um völlig in der Führung, insbesondere zwischen der inneren und der äußeren Wand, zu verschwinden. Dadurch ist ausgeschlossen, dass sich der Operateur bei dem Ausschaben des Gewebebereiches versehentlich an den Schneiden des axial zurückgezogenen Hohlkörpersegments verletzt.

In einer weiteren Ausgestaltung der Erfindung stützen sich die Federn der Schneidensegmente auf einer gemeinsamen Abstützung ab, die axial zwischen den Wänden verschiebbar ist.

Diese Maßnahme hat den Vorteil, dass sich die Schneidensegmente, sei es des gesamten Hohlkörpers oder auch nur des axial bewegbaren Hohlkörpersegments, durch die zuvor erwähnte Bewegung durch einen einzigen Verschiebevorgang hin- und herverschieben lassen, beispielsweise, um schützend in die Führung zurückgezogen zu werden.

Dies hat zusätzlich den Vorteil, dass beim Lagern oder Vorbereiten einer Operation zunächst die Schneiden hinter den distalen Rand der Vorrichtung noch zurückgezogen sind und erst zum eigentlichen Stanzvorgang entsprechend ausgeschoben werden.

Dies schützt zum einen die gegebenenfalls sehr feingliedrigen Schneidensegmente vor Beschädigung und zugleich die Handhabungsperson vor Verletzungen bei der Vorbereitung des Operationsbesteckes.

In einer weiteren Ausgestaltung weist die Vorrichtung einen Löffel zur Entnahme des ausgestanzten Gewebebereichs auf.

Diese Maßnahme hat den Vorteil, dass die Geometrie des Löffels jeweils entsprechend der Geometrie des Hohlkörpers angepasst werden kann, so dass dieser einen weiteren Beitrag zu einer optimalen Gewebeentnahme leistet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Fig. 1: einen Längsschnitt durch einen distalen Endabschnitt einer erfindungsgemäßen Vorrichtung zum Ausstanzen,
- Fig. 2: eine Draufsicht auf den distalen Endbereich der Vorrichtung von Fig. 1, wobei dessen ovale Kontur ersichtlich ist,
- Fig. 3: eine stark vergrößerte perspektivische, teilweise aufgebrochene Ansicht eines Umfangsabschnittes des distalen Endbereiches der Vorrichtung von Fig. 1,
- Fig. 4: stark schematisch den Einsatz der Vorrichtung bei einer Defektpräparation an einem Kniegelenk,
- Fig. 5: eine ausschnittsweise, stark vergrößerte Darstellung der Defektpräparation, wobei in dem mit einem Kreis umrandeten Bereich eine noch weiter vergrößerte Darstellung des Eindringens der Schneidensegmente in den hyalinen Gelenkknorpel gezeigt wird, und
- Fig. 6: eine perspektivische abschnittsweise Darstellung der erfindungsgemäßen Vorrichtung von Fig. 5 mit axial zurückgezogenem Hohlkörperelement zur Entnahme des ausgestanzten Knorpelbereichs mit einem Löffel.

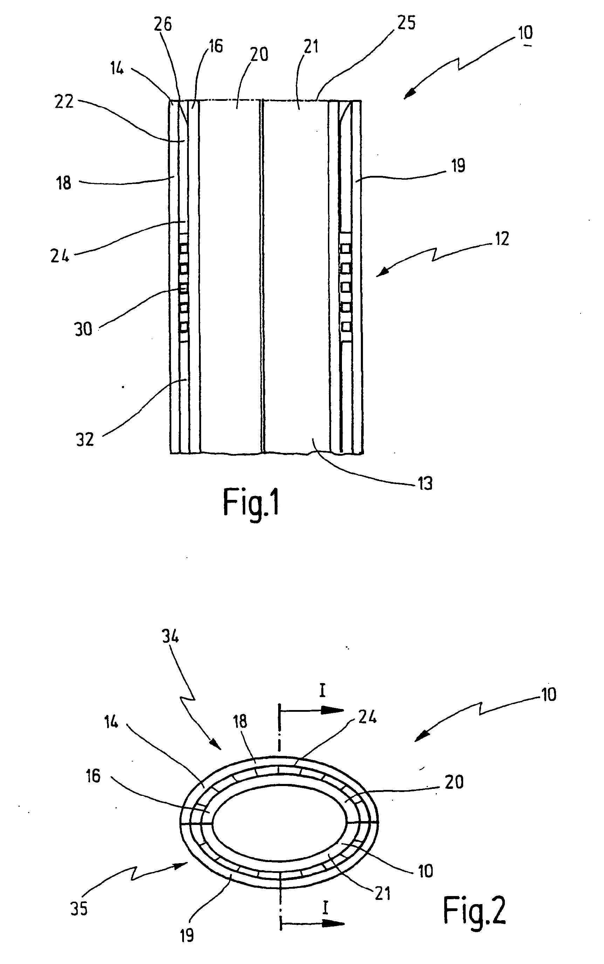

Eine in den Figuren 1 bis 6 dargestellte Vorrichtung zum Ausstanzen von Gewebebereichen ist in ihrer Gesamtheit mit der Bezugsziffer 10 bezeichnet.

Die Vorrichtung 10 weist in ihrem distalen Endbereich einen in Fig. 1 dargestellten hohlzylindrischen Körper mit elliptischem Querschnitt auf, wie das aus der Schnittdarstellung von Fig. 2 ersichtlich ist.

Der hohlzylindrische Körper 12 weist eine äußere Wand 14 und eine davon radial nach innen beabstandete innere Wand 16 auf.

Die äußere Wand 14 ist aus zwei elliptischen Halbschalen 18 und 19 zusammengesetzt, die innere Wand 16 aus entsprechenden Halbschalen 20 und 21. In dem Zwischenraum zwischen äußerer Wand 14 und innerer Wand 16 ist eine Schneide 22 aufgenommen.

Der Zusammenbau aus äußerer Wand 14 und innerer Wand 16 dient somit als eine Führung für die Schneide 22.

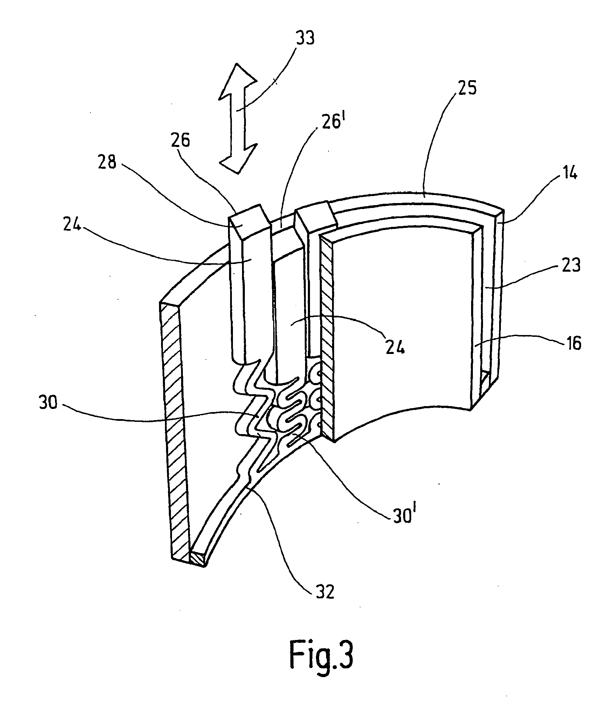

Die Schneide 22 ist, wie das insbesondere aus Fig. 3 ersichtlich ist, aus einer Vielzahl von Schneidensegmenten 24, 24' zusammengesetzt.

Jedes Schneidensegment 24 ist aus einem Hohlzylindersegment zusammengesetzt, das am distalen Ende, von radial außen nach radial innen gesehen, nach unten abfallend abgeschrägt ist. Die höherliegende Kante der Abschrägung 28 bildet somit eine Schneidekante 26.

Die Schneidensegmente 24 sind so ausgebildet und angeordnet, dass sie den Raum zwischen der äußeren 14 und der inneren Wand 16 in umfänglicher Richtung völlig ausfüllen, wobei die Schneidensegmente 24, 24' nebeneinanderliegend angeordnet sind, allerdings so, dass sie sich in axialer Richtung, wie das durch den Doppelpfeil 33 angedeutet ist, bewegen können. Aus der Draufsicht von Fig. 2 ist ersichtlich, dass insgesamt zwanzig Schneidensegmente 24 vorhanden sind.

Jedes Schneidensegment 24 ist an seinem der Schneidekante 26 gegenüberliegenden Ende mit einem elastischen Element in Form einer Feder 30 verbunden, die sich auf einer Abstützung 32 abstützt.

Jede Feder 30 ist so vorgespannt, dass sie dazu neigt, ein Schneidensegment 24 über einen distalen Rand 25 des hohlzylindrischen Körpers 12 hinaus zu drücken.

In Fig. 3 ist das Schneidensegment 24 in seiner maximalen Ausfahrposition über den distalen Rand 25 hinaus dargestellt.

Das benachbarte Schneidensegment 24 ist so weit eingefahren oder in axialer Richtung nach proximal verschoben bzw. gedrückt, dass dessen Schneidekante 26' mit dem distalen Rand 25 fluchtet.

Das in der Darstellung von Fig. 3 weiter rechts daneben liegende, nicht näher bezeichnete Schneidensegment ist in einer Zwischenstellung zwischen den beiden Stellungen der Schneidensegmente 24 und 24' dargestellt.

In der Ausführung in Fig. 3 ist die Feder 30 einerseits einstückig mit der Abstützung 32 verbunden, die wiederum eine Halbschalenform hat, andererseits integral mit dem Schneidensegment 24 verbunden. Hierbei kann diese Geometrie direkt durch einen Laserschneidevorgang aus einem Vollmaterial erfolgt sein.

Es ist auch möglich, zwischen das Schneidensegment 24 und die Abstützung 32 jeweils eine separate Feder zu legen, wobei dann entsprechende Führungen für die Feder vorgesehen sein müssen.

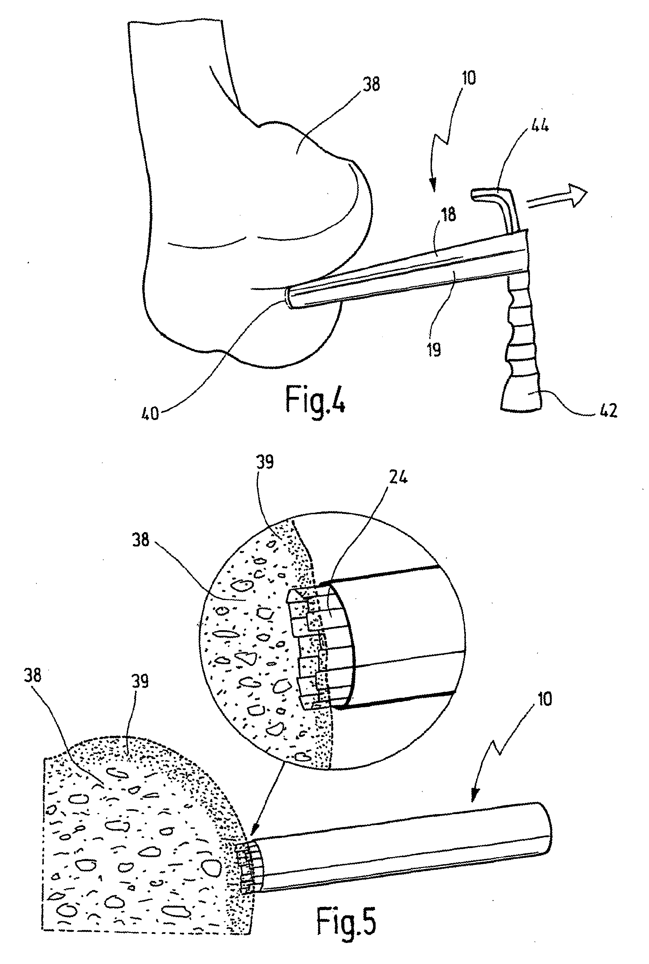

In den Figuren 4 bis 6 ist eine Handhabung der Vorrichtung 10 gezeigt, und zwar die Entnahme eines Gewebebereiches 40 eines Knorpelgewebes 39, das einen Knochen 38 bedeckt, hier beispielsweise den Oberschenkelknochen eines Menschen.

Wie eingangs erwähnt, ist der zu entnehmende Gewebebereich 40 ein Defektbereich, der durch ein anderes Gewebe ersetzt werden soll.

Die erfindungsgemäße Vorrichtung 10 wird hier als Gewebestanze eingesetzt, um diesen Gewebebereich 40 entnehmen zu können.

Wie aus Fig. 4 zu entnehmen, weist die Vorrichtung 10 zur Handhabung einen seitlich vom hohlzylindrischen Körper 12 abstehenden Griff 42 auf.

Dies ist nur ein Beispiel einer Ausführung. Wenn der Eingriff arthroskopisch vorgenommen wird, also durch einen Trokar hindurch, ist der hohlzylindrische Körper 12 entsprechend länger ausgebildet und ein Griff oder ein scherengriffartiges Handhabungselement ist in einem größeren Abstand von der Entnahmestelle angeordnet.

Dies ist aber eine Frage der jeweiligen Ausgestaltung bzw. der Art des Eingriffs, also ob minimal-invasiv oder an einem freigelegten Kniegelenk.

Der Handgriff 42 ist dabei an der Außenseite der Halbschale 19 angebracht.

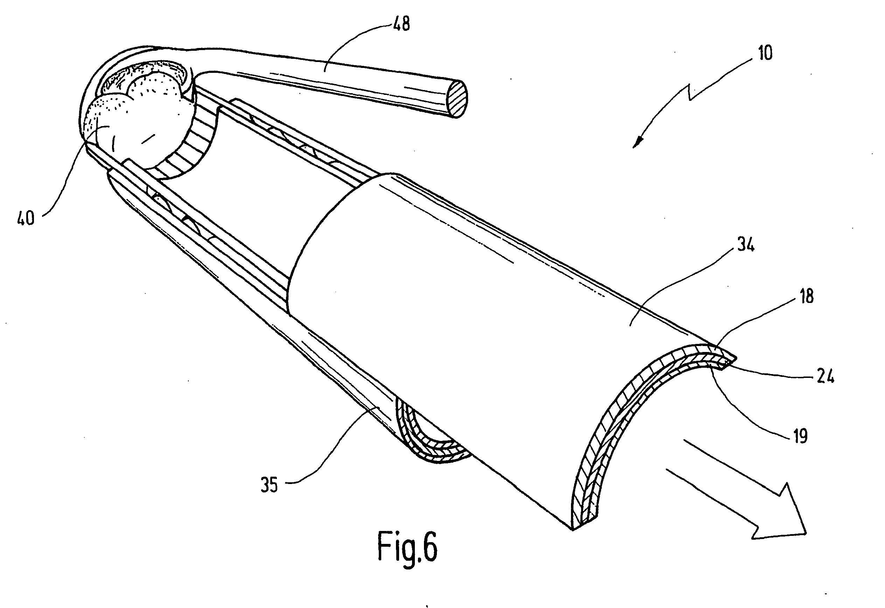

Auf der gegenüberliegenden Seite steht von der anderen Halbschale 18 eine Lasche 44 ab, deren Sinn und Zweck später im Zusammenhang mit Fig. 6 erläutert wird.

Bei der so genannten Defektpräparation wird eine Vorrichtung 10 mit der gewünschten Geometrie gewählt, hier also die Geometrie, wie sie in Fig. 2 ersichtlich ist, mit ovalem Querschnitt. Die Schneidekante 26, die sich aus der Zusammensetzung der jeweiligen Schneidekanten 26 der Schneidensegmente 24 ergibt, umgrenzt einen Bereich von etwa 10 cm². Wie insbesondere aus Fig. 5 und auch aus dem mit einem Kreis umgrenzten größeren Bereich ersichtlich ist, wird die Vorrichtung 10 mit ihrem distalen Ende an dem Knorpelgewebe 39 angesetzt, das den Knochen 38 umgibt, und zwar im Bereich des Defektes, also des Gewebebereichs 40.

Die Vorrichtung 10 wird in das Knorpelgewebe 39 eingedrückt, wobei die einzelnen Schneidensegmente 24 so weit eindringen, wie es die Oberfläche des Knochens 38 zulässt, d.h. die Kontur der Schneidekante aller Schneidensegmente 24 zusammen gesehen passt sich der entsprechend gewölbten Kontur des Knochens 38 an.

Dadurch wird jeweils im Bereich jedes einzelnen Schneidensegmentes 24 die optimale Eindringtiefe erzielt, und zugleich wird eine Defektpräparation hergestellt, bei der die Ränder sehr glatt und im rechten Winkel präpariert werden.

Zur Entnahme des ausgestanzten Gewebebereiches 40 von dem Knochen 38 wird ein in Fig. 6 dargestellter Löffel 48 mit scharfen Kanten oder eine sogenannten Kürette herangezogen.

Dabei wird der Gewebebereich bis in die osteochondrale Zone abgeschabt.

Um diesen Vorgang zu erleichtern, ist vorgesehen, dass das Hohlkörpersegment 34, wie es in Fig. 6 ersichtlich ist, in axialer Richtung nach proximal abgezogen werden kann. Die Lasche 44 erleichtert diesen Vorgang.

Das Hohlkörpersegment 34 setzt sich somit aus der Halbschale 19 der inneren Wand und der Halbschale 18 der äußeren Wand zusammen sowie den dazwischen aufgenommenen entsprechenden Schneidensegmenten 24.

Wie im Zusammenhang mit Fig. 3 erläutert, sind die Federn 30 so vorgespannt, dass sie die Schneidensegmente 24, 24' über den distalen Rand 25 hinausdrücken.

Da alle Federn 30 eines Hohlkörpersegmentes auf einer gemeinsamen Abstützung 32 abgestützt sind, eröffnet dies die Möglichkeit, diese Abstützung 32 zwischen den Halbschalen 18 und 19 noch weiter nach proximal zurückzuziehen, so dass die überstehenden Teile der Schneidensegmente 24 zwischen diesen Randbereich eingezogen werden, also nicht mehr über den distalen Rand 25 hinaus schauen. Dadurch ist ausgeschlossen, dass sich der Operateur bei einer Manipulation mit dem Löffel 48 verletzt.

Die Manipulation erfolgt derart, dass mit dem Löffel 48, wie in Fig. 6 dargestellt, der Gewebebereich 40 vollends vom Knochen abgeschabt und entnommen wird.

Je nach Operationstechnik wird anschließend in den präparierten Defektbereich entweder ein körpereigener Periostlappen, beispielsweise entnommen an der proximalen Tibea, eingesetzt, oder es wird ein entsprechend aufgezüchtetes Biopsat eingesetzt.

## Patentansprüche

1. Vorrichtung zum Ausstanzen von Gewebebereichen (40) an Knochen (38), mit einer Schneide (22), deren umlaufende Schneidekante (26) der Kontur eines auszustanzenden Gewebebereiches (40) entspricht, **dadurch gekennzeichnet, dass** die Schneide (22) aus mehreren Schneidensegmenten (24, 24') zusammengesetzt ist, die jeweils in einer axialen Richtung (33) gegen die Kraft eines elastischen Elements (30, 30') bewegbar sind.

2. Vorrichtung nach Anspruch 1, **dadurch gekennzeichnet, dass** das elastische Element als Feder (30, 30') ausgebildet ist.

3. Vorrichtung nach Anspruch 1 oder 2, **dadurch gekennzeichnet, dass** die Schneidensegmente (24, 24') in einer Führung (23) aufgenommen sind.

4. Vorrichtung nach einem der Ansprüche 1 bis 3, **dadurch gekennzeichnet, dass** ein Schneidensegment (24, 24') derart durch das elastische Element beaufschlagbar ist, dass dieses über einen axialen Längenabschnitt über einen distalen Rand (25) der Führung (23) herausdrückbar ist.

5. Vorrichtung nach Anspruch 3 oder 4, **dadurch gekennzeichnet, dass** die Führung (23) eine äußere Wand (14) und eine radial nach innen davon beabstandete innere Wand (16) aufweist, zwischen denen die Schneidensegmente (24, 24') aufgenommen sind.

6. Vorrichtung nach einem der Ansprüche 1 bis 5, **dadurch gekennzeichnet, dass** diese als Hohlkörper (12) aufgebaut ist, der in zumindest zwei Hohlkörpersegmente (34, 35) aufgeteilt ist, wobei zumindest ein Hohlkörpersegment (34) gegenüber einem anderen (35) axial verschiebbar ist.

7. Vorrichtung nach Anspruch 6, **dadurch gekennzeichnet, dass** ein Hohlkörpersegment (34, 35) die Führung (23) und die von diesem geführten Schneidensegmente (24, 24') aufweist.

8. Vorrichtung nach Anspruch 7, **dadurch gekennzeichnet, dass** die Schneidensegmente (24, 24') eines Hohlkörpersegmentes (34, 35) zusammen in der Führung (23) axial bewegbar sind.

9. Vorrichtung nach Anspruch 8, **dadurch gekennzeichnet, dass** sich die elastischen Elemente bzw. die Federn (30, 30') der Schneidensegmente (24, 24') sich auf einer gemeinsamen Abstützung (32) abstützen, die axial zwischen den Wänden (14, 16) verschiebbar ist.

10. Vorrichtung nach einem der Ansprüche 1 bis 9, **dadurch gekennzeichnet, dass** sie einen Löffel (48) zur Entnahme des ausgestanzten Gewebebereichs (40) aufweist.

## Claims

1. Device for punching out tissue areas (40) on bones (38), with a blade (22) whose circumferential cutting edge (26) corresponds to the contour of a tissue area (40) that is to be punched out, **characterized in that** the blade (22) is composed of a plurality of blade segments (24, 24') that are each movable in an axial direction (33) counter to the force of an elastic element (30, 30').

2. Device of claim 1, **characterized in that** the elastic element is designed as a spring (30, 30').

3. Device of claims 1 or 2, **characterized in that** the blade segments (24, 24') are received in a guide (23).

4. Device of anyone of claims 1 through 3, **characterized in that** a blade segment (24, 24') can be acted on by the elastic element in such a way that it can be pressed out by an axial length section past a distal edge (25) of the guide (23).

5. Device of claims 3 or 4, **characterized in that** the guide (23) has an outer wall (14) and, arranged radially inward from the latter, an inner wall (16), the blade segments (24, 24') being received between these walls.

6. Device of anyone of claims 1 through 5, **characterized in that** the device is designed as a hollow body (12) that is divided into at least two hollow body segments (34, 35), at least one hollow body segment (34) is axially displaceable relative to another one (35).

7. Device of claim 6, **characterized in that** a hollow body segment (34, 35) comprises the guide (23) and the blade segments (24, 24') guided by the latter.

8. Device of claim 7, **characterized in that** the blade segments (24, 24') of a hollow body segment (34, 35) are axially movable together in the guide (23).

9. Device of claim 8, **characterized in that** the elastic elements or springs (30, 30') of the blade segments (24, 24') are supported on a common support (32) that is axially displaceable between the walls (14, 16).

10. Device of anyone of claims 1 through 9, **characterized in that** the device comprises a spoon (48) for removing the tissue area (40) that has been punched out.

## Revendications

1. Dispositif pour découper des zones tissulaires (40) sur des os (38), muni d'une lame (22) dont l'arête périphérique tranchante (26) correspond au profil d'une zone tissulaire (40) devant être découpée, **caractérisé par le fait que** ladite lame (22) est composée de plusieurs segments (24, 24') respectivement mobiles, dans une direction axiale (33), en opposition à la force d'un élément élastique (30, 30').

2. Dispositif selon la revendication 1, **caractérisé par le fait que** l'élément élastique est réalisé sous la forme d'un ressort (30, 30').

3. Dispositif selon la revendication 1 ou 2, **caractérisé par le fait que** les segments (24, 24') de la lame sont logés dans un guide (23).

4. Dispositif selon l'une des revendications 1 à 3, **caractérisé par le fait qu'**un segment (24, 24') de la lame peut être sollicité, par l'élément élastique, de façon telle que ledit segment puisse être poussé, sur une région longitudinale axiale, au-delà d'un bord distal (25) du guide (23).

5. Dispositif selon la revendication 3 ou 4, **caractérisé par le fait que** le guide (23) comprend une paroi extérieure (14) et une paroi intérieure (16) espacée de cette dernière vers l'intérieur, dans le sens radial, les segments (24, 24') de la lame étant interposés entre lesdites parois.

6. Dispositif selon l'une des revendications 1 à 5, **caractérisé par le fait que** ledit dispositif est structurellement agencé comme un corps creux (12) scindé en au moins deux segments (34, 35), au moins un segment (34) dudit corps creux pouvant être animé de coulissements axiaux vis-à-vis d'un autre segment (35).

7. Dispositif selon la revendication 6, **caractérisé par le fait qu'**un segment (34, 35) du corps creux présente le guide (23) et les segments (24, 24') de la lame, guidés par ce dernier.

8. Dispositif selon la revendication 7, **caractérisé par le fait que** les segments (24, 24') de lame d'un segment (34, 35) du corps creux sont mobiles conjointement dans le guide (23), dans le sens axial.

9. Dispositif selon la revendication 8, **caractérisé par le fait que** les éléments élastiques ou les ressorts (30, 30') sont respectivement en applique sur un appui commun (32) pouvant coulisser axialement entre les parois (14, 16).

10. Dispositif selon l'une des revendications 1 à 9, **caractérisé par le fait qu'**il offre une curette (48) destinée à prélever la zone tissulaire (40) découpée.
